# FASCICULE DE BREVET EUROPEEN

(11) **EP 0 920 323 B1**
(45) Date de publication et mention de la délivrance du brevet: **23.07.2003**
(21) Numéro de dépôt: 98924378.7
(22) Date de dépôt: 05.05.1998
(51) Int. Cl.: A61K 35/30, A61K 35/54, A23L 1/30

(54) **NOUVELLE UTILISATION DE PHOSPHOLIPIDES D'ORIGINE ANIMALE EN THERAPEUTIQUE ET/OU DIETETIQUE**
NEUE VERWENDUNG VON PHOSPHOLIPIDEN TIERISCHEN URSPRUNGS IN DER THERAPIE UND/ODER SCHONKOST
NOVEL USE OF PHOSPHOLIPIDS OF ANIMAL ORIGIN IN THERAPY AND/OR DIETETICS

(30) Priorité: 06.05.1997 FR 9705582
(43) Date de publication de la demande: 09.06.1999
(73) Titulaire: Institut de Recherche Biologique S.A., 78340 Les Clayes-sous-Bois (FR)
(72) Inventeur: PONROY, Yves, CH-1820 Montreux (CH)
(74) Mandataire: Burtin, Jean-François
(86) Numéro de dépôt international: FR9800900
(87) Numéro de publication internationale: WO98050052

(56) Documents cités:
- EP-A- 0 502 765
- EP-A- 0 502 766
- WO-A-96/00016
- WO-A-96/00077
- US-A- 5 449 683

## Description

La présente invention se rapporte à une nouvelle utilisation de phospholipides d'origine animale en thérapeutique et/ou diététique.

La présente invention a plus particulièrement pour objet l'utilisation de phospholipides riches en acides gras polyinsaturés, en vue de la réalisation d'une composition pharmaceutique et/ou diététique ayant une action sur la sécrétion de la mélatonine.

L'invention repose plus particulièrement sur l'utilisation de phospholipides riches en acides gras polyinsaturés à longue chaîne provenant de cervelles animales ou d'oeufs de poules en vue de la réalisation d'une composition diététique permettant d'améliorer la vigilance durant le jour et les performances psychomotrices et d'apprentissage des mauvais dormeurs.

Des études antérieures ont suggéré que la mélatonine, une neurohormone produite par l'épiphyse (glande pinéale), agit comme agent de synchronisation endogène chez les humains (Armstrong - Melatonin : The internal Zeitgeber of Mammals. Pineal Res. Rev. 7 : 157 - 202, 1989).
Les propriétés pharmacologiques connues de la mélatonine sont notamment son action en tant que décontracturant musculaire, calmant, hypnotique et tranquillisant (Sugden. D, Psychopharmacological effects of melatonin in mouse and rat - J. Pharmacol. Exp. Ther. 227, 587-591, 1983).
La mélatonine permet de soulager le déficit immun induit par le stress (Maestroni Melatonin, Stress and the immune system. Pineal Res. Rev. 7, 203-206, 1989) et peut prolonger l'espérance de vie, notamment chez la souris.
La mélatonine et son principal métabolite hépatique - la 6-hydroxy mélatonine-permettent également d'éviter la lipoperoxydation expérimentale des membranes (R.J. Reiter, Antioxydant capacity of melatonin : a novel action not requiring a receptor - Neuroendocrinol, Lett. 15, 103-116, 1993).
Les dernières années, de nombreux chercheurs ont impliqué l'épiphyse et la mélatonine à la fois dans les processus du vieillissement et des maladies liées au vieillissement. Ces théories proviennent du fait de l'importance de la mélatonine dans un grand nombre de fonctions biologiques et du fait que la production de mélatonine dans l'organisme diminue peu à peu durant la vie (R.J. Reiter - The pineal gland and melatonin in relation to aging ; Experim. Geront. 30, 199-212. 1995).

Plusieurs essais cliniques ont été conduits afin de confirmer l'efficacité de la mélatonine chez les humains (R. Wurtman, Effects of melatonin on human mood and performance, Brain Research 323, 201-207, 1984 ; R. Brook, A double blind trial of melatonin as a treatment for jet lag, Biol. psychiatry 33, 526-530, 1993).
D'autres études ont également permis de vérifier que la mélatonine pouvait avoir une action sur la maladie d'Alzheimer (C.P. Maurizi, The mystery of A's D. and its prevention by melatonin).

L'utilisation chronique de mélatonine peut être discutée même si cette hormone semble sans danger. Il est néanmoins intéressant d'étudier la régulation de la production de mélatonine par l'épiphyse, notamment dans l'alimentation.
Il a été montré que la teneur en acides gras de la série n-3 de l'épiphyse peut être fortement diminuée lorsque les animaux sont nourris avec un régime déficient en acide α-linolénique (18 : 3 n-3).
Le changement le plus important a lieu pour les phospholipides glycérylés dans lesquels le niveau en acides gras de la série 22 : 6 n-3 a été réduit de 10,14 % à 2,33 %. Dans ces conditions, l'activité de l'épiphyse cultivée du rat est inférieure en terme de libération de mélatonine, dans le milieu (N.Sarda, Effect of a n-3 fatty acid-deficient diet in melatonin release in cultured rat pineal. J. of Neurochemistry 61 ; 1057-1063, 1993).

L'acide arachidonique (20 : 4 n-6) et l'acide docosahexaénoïque ou DHA (22 : 6 n-3) sont les principaux acides gras polyinsaturés de l'épiphyse. Ces deux acides gras représentent environ 25 % du total des lipides. Ils peuvent exister en outre sous forme de triglycérides dans les huiles de poisson ou sous forme de phospholipides d'origine animale provenant de cervelles ou d'oeufs.

Différentes expériences ont prouvé que les phospholipides d'origine animale constituent les meilleurs compléments alimentaires de DHA et d'acide arachidonique pour beaucoup d'usages, et les phospholipides eux mêmes sont les constituants des membranes biologiques.

Ainsi, le demandeur a déjà décrit dans ses brevets antérieurs, EP 502 766, EP 502 765, FR 2 714 574, et dans les demandes de brevets EP 95 923 373 et EP 95 923 374, des phospholipides utilisés à des fins diététiques ou thérapeutiques.

La présente invention a pour objet l'utilisation de phospholipides riches en acides gras polyinsaturés à longue chaîne provenant de cervelles animales ou d'oeufs de poules en vue de la réalisation d'une composition pharmaceutique et/ou diététique destinée à réguler la sécrétion de mélatonine.

Les acides gras polyinsaturés à longue chaîne sont ceux que l'on retrouve principalement dans l'épiphyse, à savoir l'acide arachidonique (20 : 4 n-6) et l'acide docosahexaénoïque ou DHA (22 : 6 n-3).

Les phospholipides sont associés à des excipients, des diluants ou des supports inertes, non toxiques, pharmaceutiquement acceptables en vue de la réalisation d'une composition pharmaceutique et/ou diététique permettant leur administration par voie orale ou par voie parentérale.

Les excipients ou diluants appropriés pour ces voies peuvent être des produits minéraux inertes comme par exemple le carbonate de calcium, le phosphate tricalcique, le phosphate de magnésium, l'alumine, la silice colloïdale, le kaolin, les argiles, le silicate d'aluminium, le silicate de calcium ou l'oxyde de fer pour la voie orale, l'eau ou les liquides aqueux pour la voie parentérale.

Les supports inertes peuvent être de nature organique comme les amidons, les dextrines, le lactose, la cellulose, les dérivés synthétiques de cellulose, les alginates, les carraghénates, les caséinates, les acides gras, les cires ou les résines.

Les phospholipides peuvent encore être associés à d'autres principes actifs d'action complémentaire comme les vitamines, les oligo-éléments ou les sels minéraux.

Les vitamines peuvent être celles du groupe B comme par exemple la vitamine B1, la vitamine B2, la vitamine B6, l'acide folique, l'acide pantothénique, l'acide dihydrofolique, la vitamine PP. Les oligo-éléments sont par exemple le sélénium, le lithium ou le rubidium et les sels minéraux seront par exemple les sels de magnésium.

Les compositions selon l'invention se présentent sous forme d'ampoules buvables, de flacons, de gélules, de capsules, de comprimés nus ou enrobés, de tablettes, de pastilles, de granulés ou de poudres aromatisées ou non, édulcorées ou non.
Les compositions peuvent aussi se présenter sous forme liquide comme par exemple des préparations gélifiées ou des suspensions buvables ou bien encore des émulsions huile dans l'eau.

Les phospholipides sont administrés en une quantité allant de 10 à 300 mg par prise unitaire.

Les phospholipides de la présente invention sont utilisés en vue de la réalisation d'une composition pharmaceutique et/ou diététique permettant d'améliorer la qualité du sommeil pendant la nuit, la vigilance durant le jour, et les capacités de mémoire et d'apprentissage.

Les exemples et l'étude ci-après illustrent l'invention sans toutefois la limiter.

### EXEMPLE I

Préparation d'une composition à base de phospholipides riches en acide arachidonique et en DHA, extraits de cerveaux de mammifères
- phospholipides d'origine cérébrale 10 à 300 mg
- concentré d'huile de germe de blé 10 à 100 mg
- lactose 50 à 200 mg
- phosphate tricalcique 10 à 100 mg
- bromure de magnésium 15 à 50 mg
- caséinates 5 à 20 mg
pour une gélule

### EXEMPLE II

Gélules à base de phospholipides cérébraux riches en acide arachidonique et en DHA.
- phospholipides cérébraux 10 à 300 mg
- vitamine B1 0,7 à 4,2 mg
- vitamine B2 0,8 à 4,8 mg
- vitamine B6 0,5 à 5 mg
- acide folique 100 à 600 µg
- lactose 50 à 200 mg
- stéarate de magnésium 5 à 30 mg
- silice colloïdale 5 à 25 mg
pour une gélule

L'étude suivante démontre l'application thérapeutique des phospholipides d'origine animale selon l'invention, à savoir la régulation de la sécrétion de mélatonine.

### ETUDE PHARMACOLOGIQUE ET APPLICATIONS THERAPEUTIQUES DES PHOSPHOLIPIDES SELON L'INVENTION

### 1 - Analyses

Les phospholipides d'origine animale sont extraits, à l'aide de solvants appropriés, soit de cervelles de porc, soit d'oeufs de poules qui ont été nourries à l'aide d'un régime spécial riche en acides gras de la série n-3.

Les conditions d'extraction des phospholipides ont été décrites respectivement dans les demandes de brevet européen n° 95 923 373 (extraction à partir d'oeufs de poules) et n° 95 923 374 (extraction à partir de cervelles de porc).

es produits finaux contiennent les classes usuelles de phospholipides ; cependant les proportions de ces différents phospholipides varient selon leur origine.

| Classes de phospholipides | Phospholipides cérébraux | Phospholipides d'oeufs |
|---|---|---|
| Sphingomyéline | 5 à 10 % | |
| Phosphatidylcholine | 20 à 30 % | 70 % |
| Phosphatidyisérine | 15 à 20 % | 5 % |
| Phosphatidyfinositol | 3 à 5 % | |
| Phosphatidyléthanolamine | 30 à 40 % | 16 % |
| Acide phosphatidique | 3 à 5 % | 2 % |
| Lysophospholipides | 2 à 10 % | 2 à 5 % |

Lorsque les poules ont été nourries avec un régime normal, la teneur en acides gras est quasiment identique entre les phospholipides cérébraux et les phospholipides d'oeufs de poules (voir tableau ci-dessous).

| Acides gras | Phospholipides cérébraux | Phospholipides d'oeufs |
|---|---|---|
| Acides gras saturés | 37,5 % | 40 % |
| Acides gras monoinsaturés | 37,6 % | 30 % |
| Acides gras polyinsaturés (n-6) | 15 % | 19 % |
| Acide arachidonique | (4-5 %) | (4-5 %) |
| Acides gras polyinsaturés (n-3) | 10 % | 9 % |
| Acide eïcosapentaénoïque (EPA) | (0,3 %) | (0,4 %) |
| Acide docosahexaénoïque (DHA) | (9 %) | (8 %) |

### 2 - Expérimentation animale

### Protocole expérimental

### a) Animaux et régimes subis par ces animaux

4 groupes d'animaux ont été étudiés :
- Groupes 1 et 2 :
   Les groupes 1 et 2 sont constitués par des rats de souche Wistar. Ces rats ont été nourris pendant deux générations avec un régime contenant 6 % d'un mélange (60/40) d'arachide et de graines de colza (ce groupe est dénommé « groupe témoin » ou « graines de colza ») ou avec seulement de l'huile d'arachide (ce groupe est dénommé « groupe déficient en n-3 » ou « arachide »).
   Le régime du groupe témoin est un régime équilibré en acides gras 18 : 2 n-6 et 18 : 3 n-3.
- Groupes 3 et 4 :
   Les groupes 3 et 4 sont constitués par des rats âgés de 21 jours, déjà sevrés, dont les mères font partie du groupe témoin (« graines de colza ») ou du groupe déficient en n-3 (« arachide »). Ces rats ont reçu un régime supplémenté en phospholipides animaux (« PL ») et enrichi en acides gras n-3 de façon à obtenir 200 mg d'acides gras n-3 pour 100 g d'aliments pour les animaux déficients (ce groupe est dénommé - groupe déficient en n-3 + PL - ou - « arachide » + PL -) ; 400 mg d'acides gras n-3 pour 100 g d'aliments pour des rats témoins (ce groupe est dénommé - groupe témoin + PL - ou - « graines de colza » + PL -).
   Les rats ont été nourris avec ce régime jusqu'à la fin de l'expérience.

### b) Echantillonage d'urines

Les animaux sont gardés sous strictes conditions (lumière tamisée ; température de 21 ± 1°C ; nourriture et boisson à volonté) dès qu'ils arrivent dans les cages à métabolisme. Le stress dû à l'isolation est pris en compte pendant 5 jours : ainsi, aucun échantillon ne sera prélevé durant cette période, mais cependant la reproductibilité du volume urinaire est contrôlée. Une fois cette période de 5 jours écoulée, les échantillons d'urines sont prélevés pour chaque rat.

### c) Résultats

Le dosage en acides gras de l'épiphyse met en évidence des différences significatives entre les différents groupes en ce qui concerne les acides gras de la série n-3 et spécialement pour le DHA.

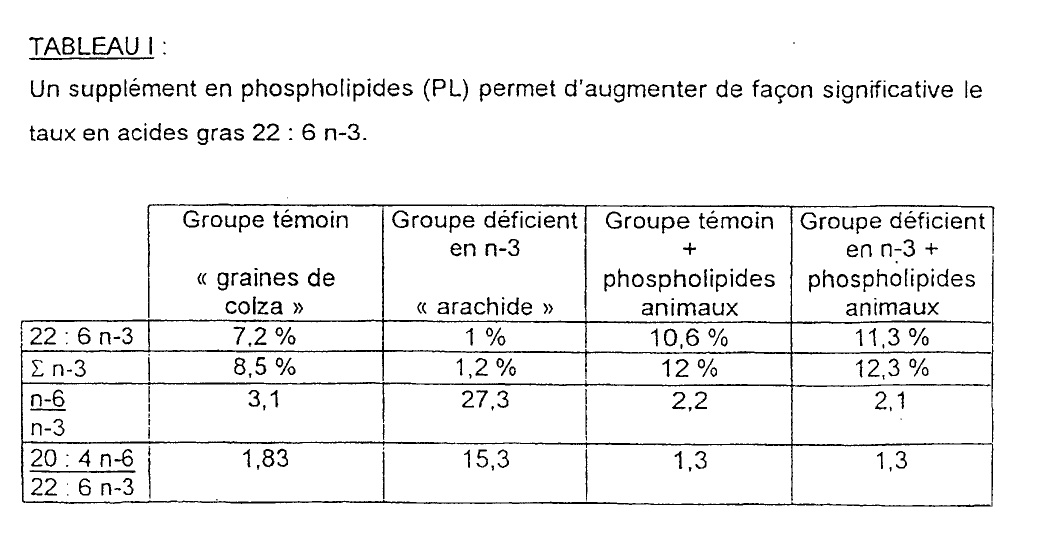

Chez ces animaux, on constate que la libération nocturne de mélatonine décroît de 32 % dans le groupe déficient en n-3 par rapport au groupe témoin.
Inversement, la prise de phospholipides d'origine animale fait augmenter de 75,8 % la mélatonine présente dans l'urine par rapport au groupe déficient en n-3 et 31 % par rapport au groupe témoin.

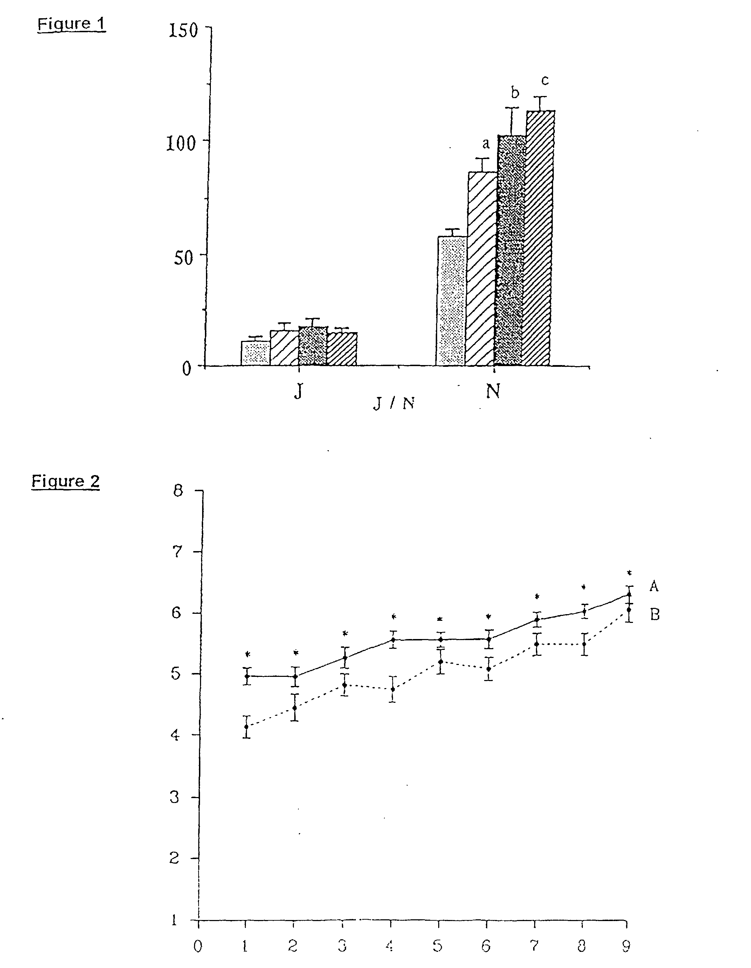

La figure 1 illustre les effets de différents régimes nutritionnels sur l'excrétion de sulfatoxy-mélatonine dans l'urine de rat pendant les phases diurnes (J) et nocturnes (N) pendant 5 semaines sur 2 nuits consécutives.

L'axe des abscisses représente le rapport (J/N), l'axe des ordonnées représente la quantité en ng de sulfatoxy mélatonine excrétée sur 12 heures.

Les rectangles gris pointillés 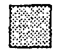 représentent le groupe « arachide » déficient en n-3, les rectangles faiblement hachurés 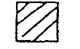 représentent le groupe témoin « graines de colza », les rectangles noirs pointillés 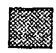 représentent le groupe « arachide » déficient en n-3 supplémenté en phospholipides animaux, et les rectangles fortement hachurés 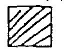 représentent le groupe témoin « graines de colza » supplémenté en phospholipides animaux.
(a) : p < 0,01 « graines de colza » par rapport à « arachide »
(b) : p < 0,01 « arachide » + PL par rapport à « arachide »
(c) : p < 0,01 « graines de colza » + PL par rapport à « graines de colza »

### d) Conclusion

Ces résultats montrent d'une part que la prise de phospholipides permet de rectifier la déficience en acides gras de la série n-3 au niveau de l'épiphyse et, d'autre part, que la prise de phospholipides riches en acides gras polyinsaturés permet de stimuler la production de mélatonine.

### 3 - Etude des effets des phospholipides cérébraux sur les mauvais dormeurs

### a) Conditions expérimentales

L'expérience suivante a été conduite sur 24 sujets sains des deux sexes, mauvais dormeurs, d'une moyenne d'âge de 30,3 ans.
Ces sujets ont dû répondre à au moins 2 critères sur les 4 critères suivants :
- temps d'endormissement : ≥ 20 minutes
- périodes d'éveil nocturne : ≥ 60 minutes
- nombre d'éveils nocturnes : 4
- durée du sommeil : ≤ 6h30

Ces critères ont été mis en évidence durant deux enregistrements polygraphiques au cours de deux nuits consécutives. Ces enregistrements polygraphiques ont été chiffrés visuellement selon les critères de Rechtschaffen et Kale.

L'étude en parallèle menée en double aveugle a duré 9 semaines à la dose journalière de 4 gélules et avec deux groupes qui ont été divisés de façon égale selon la table de randomisation de Cochran et Cox.
Groupe A : 15 mg de phospholipides cérébraux par gélule
Groupe B : placebo

Chaque sujet a rempli un agenda journalier sur son sommeil selon 3 échelles analogiques relatives au temps d'endormissement, à la vigilance lors du réveil, à la durée du sommeil, et a finalement répondu au questionnaire de l'hôpital.

### b) Résultats

- Les sujets du groupe A ont trouvé que leur sommeil a été plus profond que les sujets du groupe B.
   La figure 2 illustre la profondeur du sommeil des groupes A et B.
   L'axe des abscisses représente le nombre de semaines et l'axe des ordonnées la profondeur du sommeil.
- Les sujets du groupe A ont noté qu'ils se réveillaient moins souvent (entre 0 et 1 fois) que les sujets du groupe B (entre 1 et 2 fois).

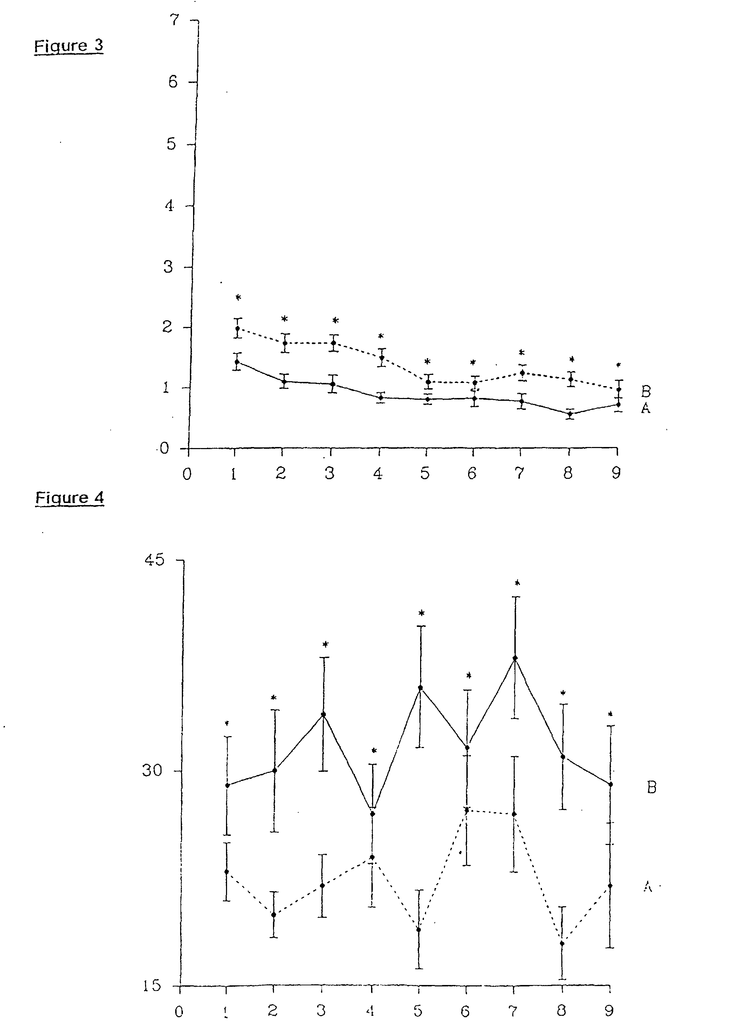

   La figure 3 illustre le nombre d'éveils nocturnes des groupes A et B.
   L'axe des abcisses représente le nombre de semaines et celui des ordonnées le nombre d'éveils nocturnes.
- Les sujets du groupe A ont également noté qu'ils se réveillaient la nuit pendant une durée moins longue que les sujets du groupe B.
   La figure 4 illustre la durée des éveils nocturnes des groupes A et B.
   L'axe des abscisses représente le nombre de semaines et celui des ordonnées la durée des éveils nocturnes (en minutes).
- La durée totale du temps de sommeil a augmenté de façon significative dans le groupe A (445 minutes ± 29 minutes de temps de sommeil) par rapport au groupe B (362 minutes ± 28 minutes de temps de sommeil).

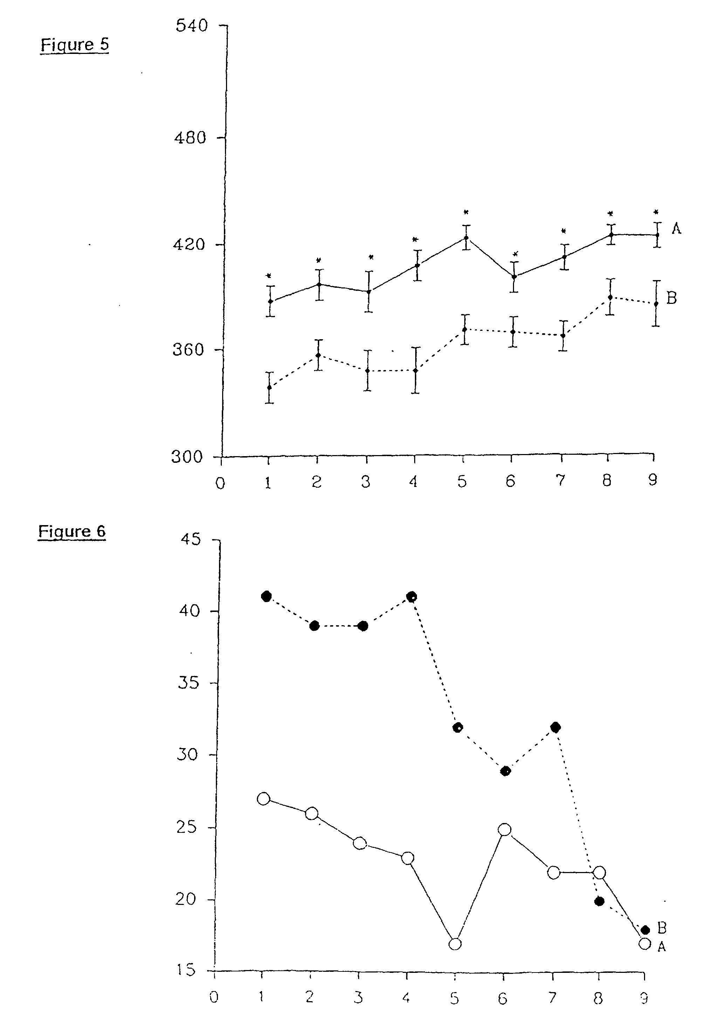

   La figure 5 illustre la durée totale du temps de sommeil des deux groupes A et B.
   L'axe des abscisses représente le nombre de semaines et celui des ordonnées la durée totale du temps de sommeil (en minutes).
- Le nombre d'épisodes de somnolence et de sieste durant la journée est plus important dans le groupe B par rapport au groupe A.
   La figure 6 illustre le nombre d'épisodes de somnolence des groupes A et B.
   L'axe des abscisses représente le nombre de semaines, celui des ordonnées le nombre d'épisodes de somnolence.

### c) Conclusion

Ces résultats démontrent l'effet favorable des phospholipides cérébraux sur la qualité et la durée du sommeil ainsi que sur la vigilance journalière.

### 4 - Etude de jour des performances psychomotrices des mauvais dormeurs

### a) Conditions expérimentales

Cette étude a été conduite dans un hôpital universitaire spécialisé dans le sommeil. 16 mauvais dormeurs ont été sélectionnés pour l'étude et ont été divisés en deux groupes parfaitement homogènes.

On a administré à chacun des deux groupes 4 capsules par jour durant 2 mois de produit A (15 mg de phospholipides cérébraux par capsule) ou de produit B (placebo).
Les sujets ont été soumis à une série de tests psychomoteurs aux jours 1, 15, 43 et 57.
Les données ont été évaluées statistiquement selon l'analyse de variance (ANOVA).

### b) Résultats

- Test d'attention concentrée (assistance par ordinateur).
   Le temps de réaction moyen a augmenté de façon significative dans le groupe A.
   Le nombre d'erreurs a augmenté de façon significative (p=0.0005) dans le groupe B (4.58) par rapport au groupe A (2.48).

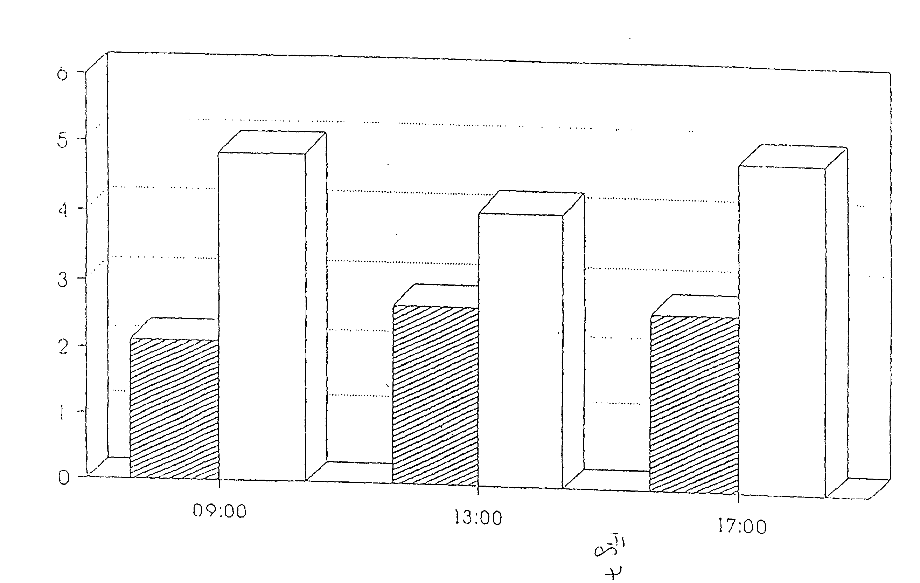

   La figure 7 illustre le test d'attention concentrée.
   Les rectangles hachurés représentent le groupe A et les rectangles blancs le groupe B (placebo).
   L'axe des abscisses représente l'heure à laquelle le test a été effectué et l'axe des ordonnées le nombre d'erreurs.
- Test de dextérité manuelle
   Ce test démontre que les phospholipides cérébraux permettent d'améliorer le test le plus complexe de coordination visio-motrice (assemblage) mais n'ont pas d'efficacité sur les tests les plus simples.
- Test des signes croisés
   La proportion de signes correctement croisés a augmenté de façon significative dans le groupe A, sans aucun effet sur les performances de rapidité.
- Test d'apprentissage verbal auditif
   Le nombre de mots mémorisés est plus grand dans le groupe A (79,96 %) que dans le groupe B (75,64 %).
   - La vitesse d'apprentissage est beaucoup plus grande dans le groupe A (4.76) que dans le groupe B (5.54).

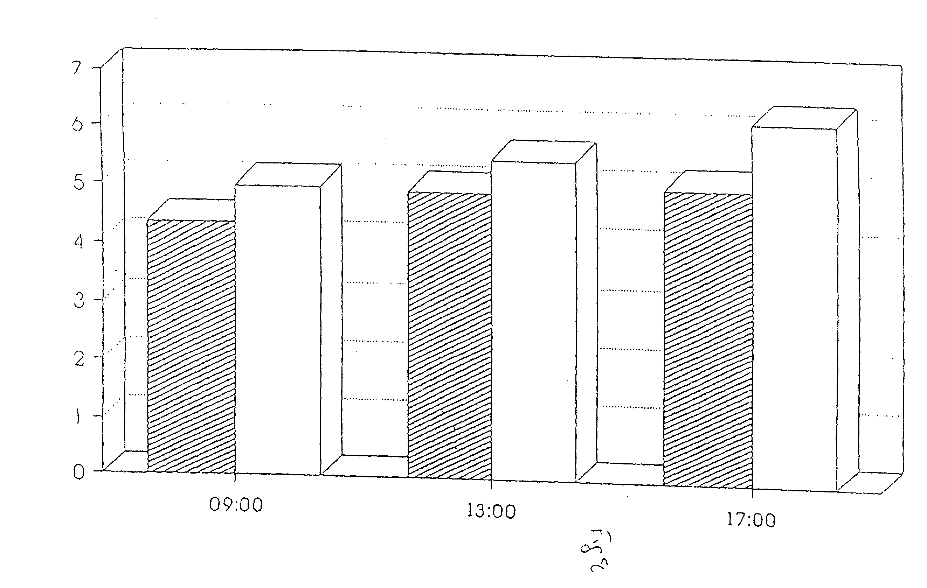

      La figure 8 illustre la vitesse d'apprentissage des groupes A et B.
      Le groupe A est représenté par les rectangles hachurés, et le groupe B (placebo) par les rectangles blancs.
      L'axe des abscisses représente l'heure à laquelle le test a été effectué et l'axe des ordonnées la vitesse d'apprentissage.
   - Le nombre d'erreurs décroît de façon significative dans le groupe A (0.56) par rapport au groupe B (1.82).

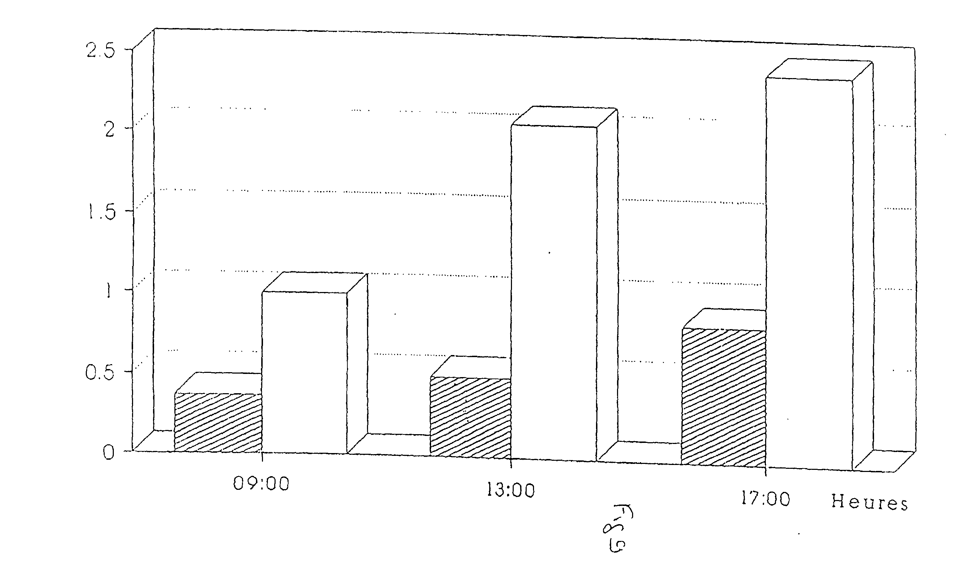

      La figure 9 illustre le nombre d'erreurs des groupes A et B.
      Le groupe A est représenté par les rectangles hachurés et le groupe B (placebo) par les rectangles blancs.
      L'axe des abscisses représente l'heure du test et celui des ordonnées le nombre d'erreurs.

Ces résultats permettent de conclure que les phospholipides cérébraux entraînent une amélioration importante des capacités d'apprentissage et de mémoire, liée à une sécrétion plus importante de mélatonine.
- Test de série numérique
   Ce test consiste à répéter une série de figures aussi longtemps que possible.
   Il n'y a pas eu de différence significative entre les 2 groupes.
- Test de reconnaissance visuelle différée
   Le nombre d'objets reconnus n'est pas différent entre les 2 groupes.

On peut en conclure que la prise de phospholipides cérébraux améliore les capacités de mémoire relatives à l'apprentissage et à la mémorisation.
Ces résultats sont en accord avec l'étude précédente qui a montré une amélioration du sommeil et de la vigilance durant le jour.
Ces résultats permettent de comprendre les effets sur les capacités d'apprentissage.

Ces études expérimentales confirment que la prise de phospholipides riches en DHA et en acide arachidonique provenant de cerveaux de mammifères ou d'oeufs de poules correctement nourries, entraîne la sécrétion de mélatonine, ce qui permet d'améliorer d'une part la qualité du sommeil et d'autre part les capacités de mémoire.

Les compositions pharmaceutiques et/ou diététiques de la présente invention, destinées à réguler la sécrétion de mélatonine, sont particulièrement adaptées pour les personnes âgées ou les mauvais dormeurs chez lesquels on observe habituellement une diminution de la concentration plasmatique en mélatonine durant la nuit.

Les compositions pharmaceutiques et/ou diététiques de la présente invention ont particulièrement des effets bénéfiques sur le sommeil et sur la qualité du sommeil, sur le réveil et la vigilance, sur l'humeur, sur les capacités d'apprentissage et de mémorisation.

L'action antioxydante de la mélatonine et son activité antiradicalaire permettent en outre d'utiliser les compositions pharmaceutiques de la présente invention pour ralentir les processus de vieillissement. La maladie d'Alzheimer est un cas particulier intéressant : dans cette maladie, la sécrétion nocturne de mélatonine est quasiment inexistante.

## Revendications

1. Utilisation de phospholipides riches en acides gras polyinsaturés à longue chaîne provenant de cervelles animales ou d'oeufs de poules en vue de la réalisation d'une composition diététique permettant d'améliorer la vigilance durant le jour et les performances psychomotrices et d'apprentissage des mauvais dormeurs.

2. Utilisation de phospholipides selon la revendication 1, dans laquelle les phospholipides sont riches en acide arachidonique (20 : 4 n-6) et en acide docosahexaénoïque ou DHA (22 : 6 n-3).

3. Utilisation de phospholipides selon la revendication 1 ou la revendication 2, **caractérisée en ce que** dans la composition diététique les phospholipides sont associés à des excipients, des diluants ou des supports inertes, non toxiques, pharmaceutiquement acceptables, permettant leur administration par voie orale ou par voie parentérale.

4. Utilisation de phospholipides selon l'une au moins des revendications 1 à 3, **caractérisée en ce que** dans la composition diététique les phospholipides sont associés à au moins une vitamine.

5. Utilisation de phospholipides selon l'une au moins des revendications 1 à 4, **caractérisée en ce que** dans la composition diététique les phospholipides sont associés à au moins un oligo-élément.

6. Utilisation de phospholipides selon l'une au moins des revendications 1 à 5, **caractérisée en ce que** dans la composition diététique les phospholipides sont associés à au moins un sel minéral.

7. Utilisation de phospholipides selon l'une des revendications précédentes dans laquelle les phospholipides sont administrés en une quantité allant de 10 à 300 mg par prise unitaire.

## Patentansprüche

1. Verwendung von Phospholipiden, die reich an langkettigen, mehrfach ungesättigten Fettsäuren sind und von Tiergehirnen oder Hühnereiern abstammen, im Hinblick auf die Herstellung einer diätischen Mixtur, die es erlaubt, die Wachsamkeit während des Tages und die psychomoforischen Fähigkeiten und die Behandlung für schlechte Schläfer zu verbessern.

2. Verwendung von Phospholipiden gemäß Anspruch 1, bei der die Phospholipide reich an Arachnidonsäure (20:4 n=6) und an Docosahexaensäure oder DHA (22: 6 n=3) sind.

3. Verwendung von Phospholipiden gemaß Anspruch 1 oder Anspruch 2, dadurch charakterisiert, dass die Phospholipide (in der diätetischen Mixtur) mit Bindemitteln, Verdünnungsmitteln oder inerten, nicht toxischen und pharmazeutisch akzeptablen Trägern vermischen sind, die eine orale oder parenterale Einnahme erlauben.

4. Verwendung von Phospholipiden gemäß wenigstens einem der Ansprüche 1 bis 3, **dadurch gekennzeichnet, dass** in der diätetischen Mixtur die Phospholipide mit wenigstens einem Vitamin vereimigt sind.

5. Verwendung von Phospholipiden gemäß wenigstens einem der Ansprüche 1 bis 4, **dadurch gekennzeichnet, dass** in der diätetischen Mixtur die Phospholipide mit wenigstens einem Oligoelement vereimigt sind.

6. Verwendung von Phospholipiden gemäß wenigstens einem der Ansprüche 1 bis 5, **dadurch gekennzeichnet, dass** in der diätetischen Mixtur die Phospholipide mit wenigstens einem Mineralsalz vereimigt sind.

7. Verwendung von Phospholipiden gemäß wenigstens einem der voranstehenden Ansprüche, bei der die Phospholipide in einer Menge von 10 bis 300 mg je Einheitsdosis verabreicht werden.

## Claims

1. Use of phospholipids reach in polyunsatured fatty acids with a long chain, stemming from animal brains or from hen eggs, with the purpose of achieving a dietetic composition allowing the improvement of alertness during the day, and of the psychomotor and learning performances in bad sleepers.

2. Use of phospholipids according to claims 1, wherein the phospholipids are reach in arachnidonic (20:4-n-6) and in docosahexaenoic acids or DHA (22:6 n-3)

3. Use of phospholipids according to claims 1 or claim 2, **characterized in that** in the dietetic composition the phospholipids are admixed with non toxic , pharmaceutically-acceptable inert excipients, diluents and carriers allowing their administration per oral or parental way.

4. Use of phospholipids according to at least of the claims 1 to 4, **characterized in that** the phospholids in the dietetic composition are associated to at least one vitamin.

5. Use of phospholipids according to at least of the claims 1 to 4, **characterized in that** the phospholids in the dietetic composition are associated to at least one oligoelement.

6. Use of phospholipids according to at least of the claims 1 to 5, **characterized in that** the phospholids in the dietetic composition, are associated to at least one mineral salt.

7. Use of phospholipids according to one of the preceding claims wherein the phospholipids are given in an amount ranging from 10 to 300 mg per unit dosage
